# EUROPEAN PATENT SPECIFICATION

(11) **EP 1 652 845 B1**
(45) Date of publication and mention of the grant of the patent: **14.11.2007**
(21) Application number: 04022287.9
(22) Date of filing: 20.09.2004
(51) Int. Cl.: C07D 307/32, C07D 307/94

(54) **Process for preparing lactones and the use thereof as odoriferous material**
Verfahren zur Herstellung von Lactonen und deren Verwendung als Geruchsstoffe
Procédé pour la préparation des lactones et leur utilisation comme substances odorantes

(43) Date of publication of application: 03.05.2006
(73) Proprietor: KAO CORPORATION, Chuo-ku Tokyo (JP)
(72) Inventor: Markert, Thomas, Dr., 40789 Monheim (DE); Porrmann, Volker, 40723 Hilden (DE); ten Pierik, Theo, 05916L Venlo (NL)
(74) Representative: HOFFMANN EITLE

(56) References cited:
- WO-A-01/42191
- US-A- 2 683 721
- US-A- 2 957 805
- DENIS M. BAILEY, ROBERT E. JOHNSON: "Reduction of Cyclic Anhydrides with NaBH4. Versatile Lactone Synthesis" J. ORG. CHEM., vol. 35, no. 10, 1970, pages 3574-3576, XP002367068
- A.J.M. JANSEN, A.J.H. KLUNDER, B. ZWANENBURG: "Enzymatic resolution of Norbor(ne)nylmethanols in organic media and an application to the synthesis of (+)- and (-)-endo-Norbornene lactone" TETRAHEDRON, vol. 47, no. 29, 1991, pages 5513-5538, XP002367069
- SRINIVASAN NARASIMHAN: "Improved procedure for lithium borohydride reduction of cyclic anhydrides to lactones in tetrahydrofuran" HETEROCYCLES, vol. 18, 1982, pages 131-135, XP009061458
- BARRY M. TROST, CHUL BOM LEE: "Geminal Dicarboxylates as Carbonyl Surrogates for Asymmetric Synthesis. Part II. Scope and Applications" J. AM. CHEM. SOC., vol. 123, 2001, pages 3687-3696, XP002367070
- PATENT ABSTRACTS OF JAPAN vol. 2003, no. 03, 5 May 2003 (2003-05-05) & JP 2002 322489 A (SHIONO KORYO KK), 8 November 2002 (2002-11-08)
- PATENT ABSTRACTS OF JAPAN vol. 003, no. 114 (C-059), 21 September 1979 (1979-09-21) & JP 54 095535 A (NIPPON ZEON CO LTD), 28 July 1979 (1979-07-28)

## Description

The present patent application relates to a process of preparing lactones and the use thereof as odoriferous materials, as well as to fragrance compositions.

The preparation of some lactone derivatives as well as their use as an odoriferous material or as fragrances is already known from the literature. Although some of the known lactone derivatives have good fragrance properties, there is still a need for compounds having characteristic odour profiles of different quality.

Having regard to the processes for preparing various types of lactones, the Bayer-Villiger-oxidation is known according to which cyclic ketones are reacted with a peroxy acid such as CH₃COOOH. The addition of the oxygen of such a peroxide may be improved in the presence of a Lewis acid.

The preparation of lactones comprising an unsaturated alkyl ester group starting from e.g. bismalonate is also known (see B. Trost et al., in J. Am. Chem. Soc. 2001, 123, 3687 - 3696). This reaction is performed by using tetrabutyl ammonium fluoride in THF under reflux. This reaction results in a mixture of α-lactones, and a further dialkylation of lithium iodide under reflux conditions leads to the corresponding lactone methyl ester.

Also known is the Claisen rearrangement and the subsequent derivatisation to obtain lactones comprising an unsaturated alkyl group as a side chain.

The known processes of preparing lactones have the following disadvantages.

For performing the reactions some specified reactants are necessary such as hydrofluoric acid, methyl cyanide or potassium hexamethyl disilazane. Accordingly, it is necessary to prepare specified reagents or reducing agents. Even though the above-described reactions lead the desired lactones in good yields, it is especially disadvantageous that these reactions can only be performed on a laboratory scale, however not on a technical scale. Moreover, the reducing agents used for the known processes are toxic, inflammable or difficult to prepare. Often a low temperature such as less than -20°C must be adopted.

Accordingly, there is still a strong need for providing a simple process of preparing lactones to be adopted on a large scale, which process leads to a good yield of the desired lactones.

Accordingly, the present invention relates to a process for preparing lactones from the corresponding aldehyes in the presence of reducing agents which are non toxic and are safe to handle.

More precisely, the present patent application is related to a process of preparing lactones of the following general Formula (I) 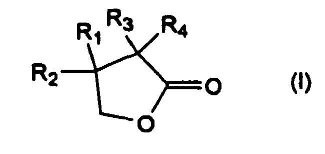 wherein R₁ and R₂ or R₃ and R₄ combine with each other to form a cyclohexenylene ring which may comprise one or two C₁₋₄ alkyl groups as a substituent and wherein the other two of these radicals each represent a hydrogen atom, the process comprising reacting an anhydride of the Formula (II) 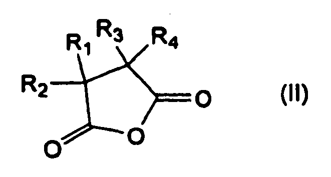 wherein the radicals R₁ to R₄ have the same meaning as defined above, with a reducing agent in an organic solvent.

According to a particularly preferred embodiment the reducing agent is lithium or sodium borohydride. As a solvent, any solvent may be used which is capable of forming a type of hydride complex with the reducing agent, and particularly preferred are alcohols, which may be monohydric alcohols or polyhydric alcohols. A particularly suitable alcohol is a lower alcohol comprising 1 to 4 carbon atoms such as methanol, ethanol, propanol (including n-propanol and iso-propanol) or butanol (including n-butanol, i-butanol and t-butanol). C₁₋₄ alkylene glycols such as ethylene glycol or propylene glycol are also suitable. A particular preference is given to methanol. Also a mixture of the solvents may be used.

The reaction may be performed at room temperature, but the temperature range may also include the range of 0°C to 40°C. Preferably, the reaction is performed under atmospheric pressure, but a reduced pressure may also be employed.

The reaction of the anhydride with the reducing agent and the solvent is performed for a proper reaction time such as approximately 0.5 to approximately 5 hours, preferably 1 to 3 hours under stirring. During the reaction, the temperature raises as a result of the exothermic reaction. If necessary, the reaction mixture may be cooled by stirring over an ice bath.

After the reaction of the components, the mixture may be allowed to continue being stirred overnight at room temperature.

After the reaction, a mixture of the different types of lactones is obtained because the anhydrides contain two sites which may react with the reducing agent. The lactones may then be further processed by means of extraction and/or washing as well as drying or concentration. Moreover, a distillation step may be performed. These latter mentioned steps are well known to the skilled person.

According to the process of the present patent application the lactones may be prepared in a simple manner without the necessity of using dangerous or toxic reagents.

The lactones obtained by the process of the present patent application are particularly suitable as odoriferous materials or in fragrance compositions. The compounds according to the present invention are fragrances having differently nuanced intense odor profiles of distinctly different qualities. They are characterized by a considerably great odor intensity and emanative power. The after-odor (evaluation after 24 hours) of the lactones produced in accordance with the present patent application, is very intensive.

The compounds produced in accordance with the process of the present invention may advantageously be used as structural elements for new fragrance compositions. Fragrance compositions contain, for example, natural, synthetic or partly synthetic fragrances, essential oils and plant extracts. The percentage in which the compounds obtained by the process of the invention, or the mixtures thereof, may be used in fragrance compositions are between 1 and 50% by weight, based on the mixture as a whole.

Compositions of this type may be used both for perfuming cosmetic preparations such as creams, lotions, toilet waters, aerosols and toilet soaps, and in alcohol-based perfumery. They may also be used for perfuming industrial products such as detergents, fabric softeners and textile treatment preparations, as well as, for example, candles such as stearin candles. For perfuming these various products, the compositions are added in concentrations of 0.05 to 2% by weight, based on the product as a whole.

The respective anhydrides may be prepared according to well known methods, for example by the ene-reaction of alkenes with maleic anhydride or by means of the known Diels-Alder-reaction (1.4-addition) of itaconic anhydride with suitable dienes such as piperylene, 2-mehtyl-1,3-pentadiene or isoprene. The resulting anhydride is then reacted with the suitable reducing agent in a solvent to produce the desired lactones.

The following examples are intended to illustrate the invention without limiting it in any way.

### Example 1:

### Reaction of isoprene with itaconic anhydride

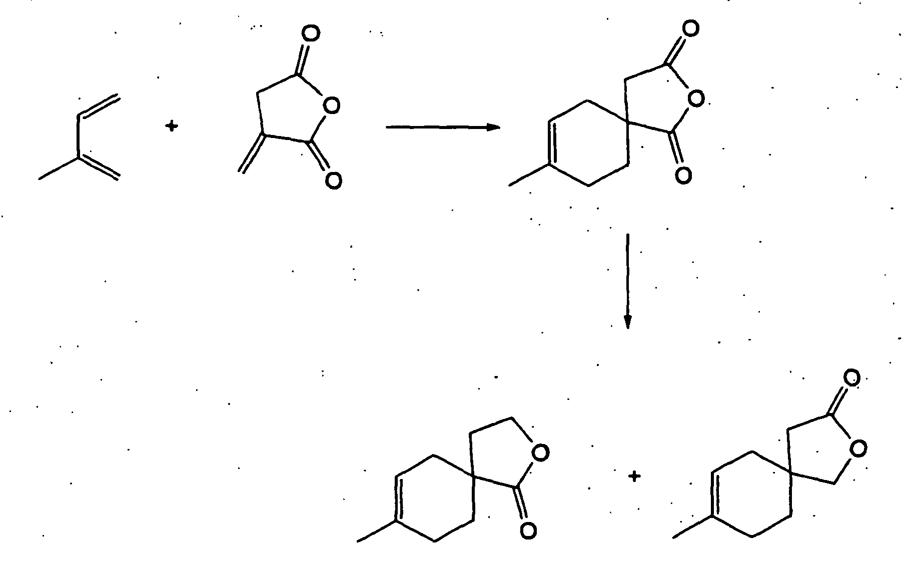

Starting materials: 34 g (0.5 mol) isoprene, 56 g (0.6 mol) itaconic anhydride, 250 ml cyclohexane

The components were charged together in an autoclave and were heated at 150°C under 50 bar and under nitrogen atmosphere for 3 hours. The analysis by using gas chromatography revealed 2.4% reactant, 9.3 and 86.1% of the products.

The reaction mixture was concentrated and 60g of the raw material was distillated in a bulb tube distillation apparatus. 40g of the distillate were obtained, which produced white crystals.

Analysis: IR-spectrum (DAT-technique) shows characteristic peaks at wavelengths of 894, 943, 989, 1054, 1120, 1164, 1230, 1778, 1840 and 2898 cm⁻¹.

The 1H-NMR-spectrum (in CDCl₃, 400 MHz) shows one metyhl group as a broad singlet at 1.7 ppm (3H). The 3 CH₂- groups within the five-membered ring show 3 signal groups at 1.8 (multiplet 1H), 2.1 (signal accumulation, 4 H) and 2.6 ppm (broad doublet, 1H). The CH₂-group of the anhydride shows a signal group at 2.8 ppm (doublet of doublet, 2H). The olefinic proton shows one broad signal at 5.4 ppm (broad multiplet, 1H).

Odor: very weak.

### Example 3:

### Preparation of 8-methyl-1-oxaspiro[4.5]dec-7-ene-2-one

38 g (0.17 mol) of an addition product of isoprene and itaconic anhydride (see Example 1); 7.6 g (0.2 mol) sodium borohydride, and 100 ml isopropanol

The sodium borohydride was suspended in 100 ml isopropanol and the anhydride mixture was added in portions at room temperature for 2 hours with strong stirring. There was a strong exothermic reaction between these two components, and the temperature of the reaction mixture increased to 33°C. A milky solution was obtained, which was stirred at room temperature overnight. The gas chromatography showed the formation of 2 main components and 2 side components.

The reaction mixture was poured onto 500 ml of an ice/hydrochloric acid mixture and was extracted with ether. The organic phases were washed until these became neutral, dried, evaporated and distillated by using a bulb tube.

17.2 g of 8-methyl-1-oxaspirol[4,5]dec-7-ene-2-one (mixture of 4 isomers) were obtained.

Analysis: The IR-spectrum (DAT-technics) shows characteristic bands at 961, 1011, 1177, 1199, 1376, 1440, 1439, 1771 (lactone-C=O- vibrations) and 2915 cm⁻¹

The 1H-NMR-spectrum (in CDCl₃, 400 MHz) shows 1 methyl group in the form of 2 broad singlets at 1.6 ppm. The CH₂-group adjacent to the ring oxygen atom shows 2 signal groups at 4.0 (singlet) and 4.25 ppm (doublet of the triplet). The C=O-group adjacent to the CH₂ shows a signal group at 2.3 ppm (doublet of doublet, intensity 1.8 H). 2 signal groups at 2.0 and 2.1 ppm correspond to the 3 CH₂-groups of the cyclohexane ring. The olefinic proton shows 2 signals at 5.3 (broad doublet, 0.8 H) and 5.4 ppm (broad doublet, 0.2 H).

Odoriferous characteristic: At the beginning dry, green, lactone, hay odor, after 24 hours on an odor strip the odor was warm , green, coconut.

### Example 3:

### 6,8-dimethyl-1-oxaspiro[4,5]dec-7-ene-2,4-dione

41 g (0,5 mol) trans-2-methyl-1,3-pentadiene, 56 g (0,6 mol) itaconic anhydride and 200 ml cyclohexane

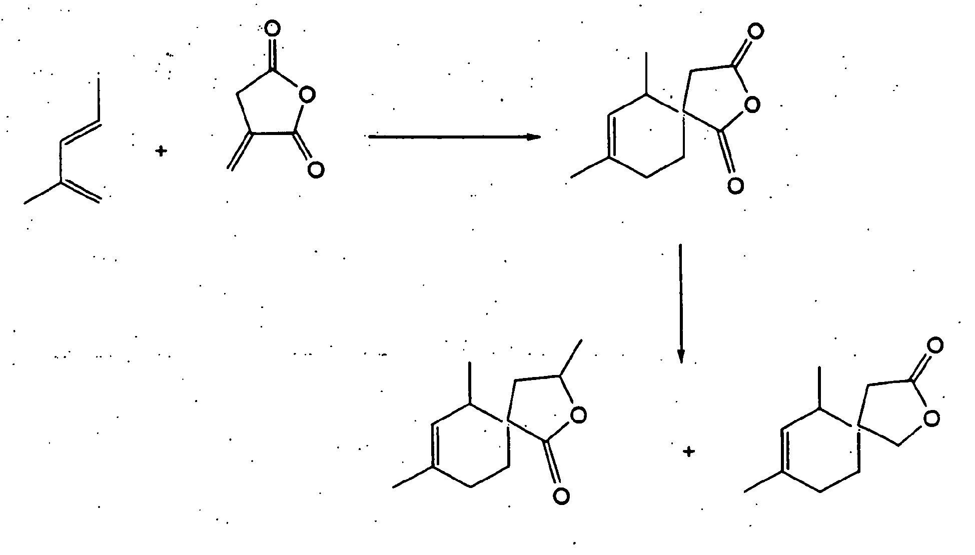

The components were charged together into an autoclave and were heated under 50 bar and under a nitrogen atmosphere for 3 hours and at 150°C. The gas chromatography showed 8% of educt and additionally 87% of the products. The reaction product was distilled by means of a bulb.tube, and 41g of white crystals were obtained.

Analysis: The IR-spectrum (DAT-technique) shows characteristic bands at 888, 912, 936, 964, 1078, 1119, 1228, 1414, 1451, 1770, 1830 and 2967 cm-¹.

The 1H-NMR-spectrum (in CDCl₃), 400 MHz) shows 1 methyl group in the form of 2 doublets at 0.95 (1,1H) and 1.1 ppm (1.9 H). The methyl group at the double bond shows 2 broad singlets at 1.7 ppm (3H). The CH₂-group within the five-membered ring shows 2 signal groups at 2.8 (singlet, 1.4H) and 2.65 ppm (doublet of doublet, 0.6 H). The two CH₂-groups within the cyclohexene ring show 6 signal groups between 1.9 and 2.3 ppm. One signal group at 1.8 ppm correspond to the CH within the cyclohexene ring which bears the methyl group (2 quadruplets). The olefinic proton shows 2 signals at 5.1 (broad singlet, 0.3 H) and 5.4 ppm (broad singlet, 0.7 H)

Odorous characteristics: odorless

### Example 4:

### Preparation of 6,8-dimethyl-1-oxaspiro[4,5]dec-7-ene-2-one

31.4 g (0.14 mol) 6,8-dimethyl-1-oxaspiro[4,5]dec-7-ene-2,4-dione (Example 3), 6.3 g (0.16 mol) sodium borohydride, 180 ml isopropanol

The 6,8-dimethyl-1-oxaspiro[4,5]dec-7-ene-2,4-dione was dissolved in 180 ml isopropanol and the sodium borohydride was added in portions at room temperature under strong stirring within 30 minutes. An exothermic reaction occurred with the two components, and the temperature of the reaction mixture increased to 55°C. A milky solution was obtained which was stirred at room temperature overnight. The gas chromatogram showed the formation of 4 main components and 2 side components.

The reaction mixture was added to 200 ml of the mixture of ice and hydrochloric acid and was extracted with ether. The organic phases were washed until these became neutral, dried, evaporated, and 39 g of the raw material were distillated by using a bulb tube.

Analysis: The IR-spectrum (DAT-technique) shows characteristic bands at 987, 1031, 1151, 1171, 1451, 1771 (lactone-C=O-vibrations) and 2917 cm⁻¹.

The 1H-NMR-spectrum (in CDCl₃, 400 MHz) shows 4 doublets (3H) at 1 ppm for the one methyl group within the cyclohexene ring and at least 2 broad singlets at 1.6 ppm for the other methyl group. The CH₂-group adjacent to the ring oxygen atom shows signal groups between 3.9 and 4.3 ppm (multiplets and doublets). The CH₂-group adjacent to the C=O-group shows signal groups between 2.1 ppm (multiplet) and 2.4 ppm (doublet of the doublet). 2 Signal groups at 1.5 and 1.7 pm and 2 ppm (strong multiplets) correspond to the 3 CH₂-groups of the cyclohexene ring. The olefinic proton shows 4 signals between 5.1 and 5.4 ppm (broad singlets 1H).

Odorous characteristic: at the beginning sweet and fruity odor, cumarin-like, after 24 hours on an odor strip the odor was warm, hay-like.

## Claims

1. Process for preparing lactones of the Formula (I) 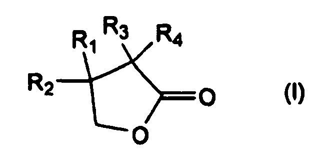 wherein R₁ and R₂ or R₃ and R₄ combine to each other to form a cyclohexenylene ring which may comprise one or two C₁₋₄ alkyl groups as a substituent and wherein the other two of these radicals each represent a hydrogen atom, the process comprising reacting an anhydride of the of the Formula (II) 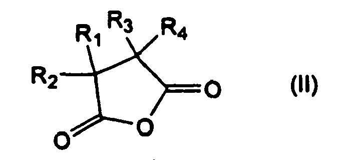 wherein the radicals R₁, R₂, R₃ and R₄ have the same meaning as defined above, with a reducing agent in an organic solvent.

2. Process as claimed in Claim 1, wherein the reducing agent is lithium or sodium boron hydride.

3. Process as defined in Claims 1 or 2, wherein the solvent is a monohydric or polyhydric alcohol.

4. Process as defined in Claim 3, wherein the alcohol is a C₁₋₆ alcohol or C₁₋₄ alkylene glycol.

5. Process as defined in any one of the Claims to 4, wherein the reaction is performed at a temperature of between 0 to 40°C, preferably at room temperature.

6. Use of the lactones obtainable by the process as defined in any one of Claims 1 to 5, as odoriferous materials.

7. Use as claimed in Claim 6, for perfuming cosmetic preparations and technical products or in alcohol-based perfumery.

8. Fragrance compositions, **characterized by** a content of a lactone derivative produced in accordance with the method as defined in any one of Claims 1 to 5, or a mixture of these in a quantity of 1 to 50% by weight, based on the composition as a whole.

## Patentansprüche

1. Verfahren zur Herstellung von Lactonen mit der Formel (I) : 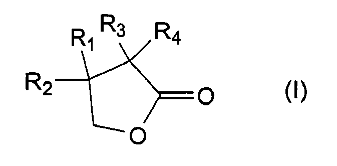 worin R₁ und R₂ oder R₃ und R₄ miteinander kombinieren, unter Bildung eines Cyclohexenylenrings, der ein oder zwei C₁₋₄-Alkylgruppen als Substituenten haben kann, und worin die anderen beiden dieser Radikale jeweils ein Wasserstoffatom bedeuten, wobei das Verfahren die Reaktion eines Anhydrids der Formel (II) : 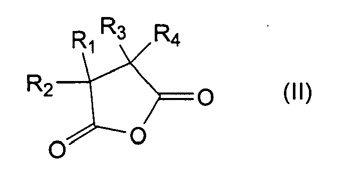 worin die Radikale R₁, R₂, R₃ und R₄ die gleichen Bedeutungen wie oben aufweisen, mit einem Reduktionsmittel in einem organischen Lösungsmittel umfaßt.

2. Verfahren nach Anspruch 1, worin das Reduktionsmittel Lithium- oder Natriumborhydrid ist.

3. Verfahren nach Anspruch 1 oder 2, worin das Lösungsmittel ein einwertiger oder mehrwertiger Alkohol ist.

4. Verfahren nach Anspruch 3, worin der Alkohol ein C₁₋₆-Alkohol oder C₁₋₄-Alkylenglycol ist.

5. Verfahren nach einem der Ansprüche 1 bis 4, worin die Reaktion bei einer Temperatur zwischen 0 und 40°C, bevorzugt bei Raumtemperatur, durchgeführt wird.

6. Verwendung der Lactone, erhältlich durch das Verfahren wie in einem der Ansprüche bis 5 definiert, als Duftmaterialien.

7. Verwendung nach Anspruch 6, zur Parfümierung von Kosmetikpräparaten und technischen Produkten oder in der Parfümerie auf Alkoholbasis.

8. Duftzusammensetzungen, **gekennzeichnet durch** einen Gehalt eines Lactonderivats, erzeugt entsprechend dem Verfahren wie in einem der Ansprüche 1 bis 5 definiert, oder eine Mischung dieser in einer Menge von 1 bis 50 Gew.%, bezogen auf die gesamte Zusammensetzung.

## Revendications

1. Procédé pour la préparation de lactones répondant à la formule (I) 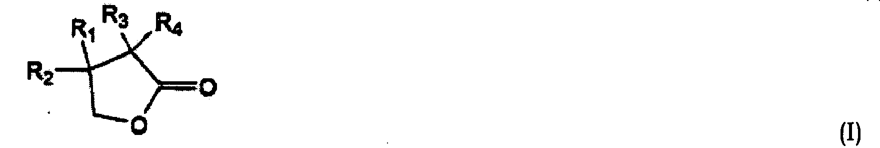 dans laquelle les radicaux R₁ et R₂ ou R₃ et R₄ sont combinés ensemble de façon à former un cycle cyclohexénylène qui peut comprendre un ou deux groupes alkyle en C₁ à C₄ en tant que substituant et dans laquelle chacun des deux autres radicaux représentent un atome d'hydrogène, le procédé comprenant la réaction d'un anhydride répondant à la formule (II) 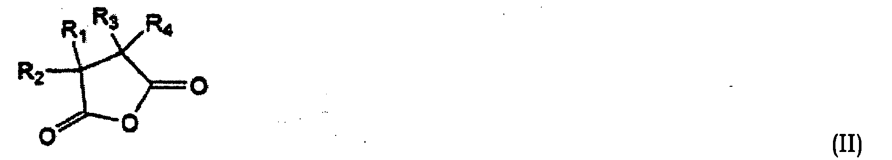 dans laquelle les radicaux R₁, R₂, R₃ et R₄ ont les mêmes significations que définies ci-dessus, avec un agent réducteur dans un solvant organique.

2. Procédé selon la revendication 1, dans lequel l'agent réducteur est l'hydrure borique de lithium ou de sodium.

3. Procédé selon les revendications 1 ou 2, dans lequel le solvant est un alcool monohydrique ou un polyol.

4. Procédé selon la revendication 3, dans lequel l'alcool est un alcool en C₁ à C₆ ou un alkylène glycol en C₁ à C₄.

5. Procédé selon l'une quelconque des revendications 1 à 4, dans lequel la réaction est effectuée à une température comprise entre 0 et 40 °C, de préférence à température ambiante.

6. Utilisation des lactones que l'on peut obtenir au moyen du procédé défini selon l'une quelconque des revendications 1 à 5, en tant que matières odorantes.

7. Utilisation selon la revendication 6, pour parfumer des préparations cosmétiques et des produits techniques ou en parfumerie à base d'alcool.

8. Compositions de parfum,
**caractérisée par**
une teneur en un dérivé lactone produit selon le procédé défini selon l'une quelconque des revendications 1 à 5, ou un mélange de celles-ci en une quantité de 1 à 50 % en poids, sur la base de la composition dans son ensemble.
